# EUROPÄISCHE PATENTSCHRIFT

(11) **EP 0 487 459 B1**
(45) Veröffentlichungstag und Bekanntmachung des Hinweises auf die Patenterteilung: **15.05.1996**
(21) Anmeldenummer: 91810910.9
(22) Anmeldetag: 22.11.1991
(51) Int. Cl.: G01N 33/543, G01N 33/58, G01N 33/555, G01N 33/80

(54) **Verfahren zum Nachweis von Antikörpern**
Procedure for detection of antibodies
Procédé pour la détection d'anticorps

(30) Priorität: 22.11.1990 CH 3697/90
(43) Veröffentlichungstag der Anmeldung: 27.05.1992
(73) Patentinhaber: Giannitsis, Dimitrios, Dr., D-76133 Karlsruhe (DE); Häcker-Shahin, Bärbel, Dr., D-68723 Schwetzingen (DE)
(72) Erfinder: Giannitsis, Dimitrios, Dr., D-76133 Karlsruhe (DE); Häcker-Shahin, Bärbel, Dr., D-68723 Schwetzingen (DE)
(74) Vertreter: Fischer, Franz Josef

(56) Entgegenhaltungen:
- EP-A- 0 058 780
- EP-A- 0 141 939
- EP-A- 0 305 337
- EP-A- 0 367 468

## Beschreibung

Die Erfindung betrifft ein Verfahren zum Nachweis von in Blutserum enthaltenen Antikörpern gegen antigene Strukturen auf Zellmembranen, insbesondere auf Thrombozytenmembranen.

Bei der Transfusion von Thrombozytenhochkonzentraten sind für den Empfänger geeignete Spender zu finden, d.h. das Vorliegen von Antikörpern gegen antigene Strukturen der Thrombozytenmembranen ist auszuschliessen, um den Erfolg der Transfusion sicherzustellen und das Komp likationsrisiko zu vermindern. In vielen Fällen muss eine solche Verträglichkeitsuntersuchung in kurzer Zeit durchführbar sein.

Es sind solche Nachweisreaktionen bekannt, welche Indikatorerythrozyten verwenden (Shibata Y. et al., Vox. Sanguin. 1981; 41: 25-31; Rachel J. et al., Med. Lact. Science 1985; 43: 194-195; Rachel J. et al., Amb. J. Clin. Pathol. 1988; 90: 63-68). Die bekannten Verfahren besitzen den Nachteil, dass die verwendeten Indikatorerythrozyten lediglich 2 bis 3 Wochen haltbar sind. Dies gilt sogar bei einer Glutaraldehydfixierung.

In der EP-A-0 058 780 ist eine Antigen/Antikörper-Bestimmungsmethode in einer Flüssigkeit beschrieben. Hierzu werden Partikel, die Antigene an der Oberfläche aufweisen, und Antikörper in der Flüssigkeit inkubiert, wobei das Antigen oder der Antikörper von bekannter Spezifität ist. Nach der Inkubation wird die Flüssigkeit in einen Behälter mit kegel- oder keilförmiger Bodenvertiefung eingebracht. Dabei ist mindestens die Vertiefung des Behälters mit einer Immunglobulin-bindenden Komponente beschichtet, welche gegen die zu bestimmenden Antikörper gerichtet ist. Nach der Inkubation wird das Gefäss einer Zentrifugation unterworfen, wobei im positiven Fall (d.h. wenn die Partikel mit antigenen Strukturen oder antikörper/antiimmunoglobulinbildenden Komponenten gebunden sind), nach einer Zentrifugation nur ein kleines Sediment gebildet wird. Im negativen Fall hingegen, d.h., wenn keine Bindung an die Strukturen, mit denen die Zellwand beschichtet ist, vorkommt, wird ein grosses Sediment gebildet. Die vorliegende Erfindung hingegen stellt ein einfacheres Verfahren zur Verfügung, worin die nachzuweisenden Antikörper gegen antigene Strukturen auf Zellmembranen direkt an die Antigene der Zellmambranen gebunden werden, die an der Gefässoberfläche fixiert sind.

EP-A-0 367 468 beschreibt ein Verfahren zur Trocknung von Säugetierzellen für ihre Verwendung als Absorbens in Festphasen-Immunoassays. Darin werden Säugetierzellen wie Erythrozyten, Lymphozyten, Leukozyten und Blutplättchen auf einem Träger immobilisiert und die Zellen anschliessend durch eine trocknende Lösung, wie beispielsweise einer wässrigen Lösung von Monosacchariden, Disacchariden, Trisacchariden, Cyclitol und Salzen getrocknet. Die Träger können Reagenzgläser, Mikrotiterplatten usw., sein. Das Verfahren erzeugt "Monolayers" von Zellen auf einem festen Träger. Die beschichteten Träger können in einem weiten Bereich von immunologischen Tests verwendet werden. Für die Herstellung der Mikrotitterplatten ist eine verhältnismässig lange Trockenzeit von in der Regel sieben Tagen erforderlich.

Der Erfindung liegt die Aufgabe zugrunde, eine schnelle und einfache Methode vorzuschlagen, die alle An tikörper gegen antigene Strukturen auf Zellmembranen, wie Thrombozytenmembranen erfasst und deren einzelne Reagenzien im Gegensatz zu den bekannten Verfahren eine lange Haltbarkeit zeigen, so dass die Testreagenzien über Monate hinweg gelagert und somit vorrätig gehalten werden können.

Ebenso soll das Herstellungsverfahren durch Schritte möglich sein, die verhältnismässig mit kurzen Behandlungszeiten möglich sind.

Gegenstand der vorliegenden Erfindung ist demzufolge das im Anspruchsteil beschriebene Verfahren zum Nachweis von Antikörpern gegen antigene Strukturen auf Thrombozytenmembranen und anderen Zellmembranen.

Der erfindungsgemässe Test ist ausserdem dazu geeignet, Patientenantikörper gegen Thrombozytenmembran-Antigene näher zu definieren, wenn die zum Test eingesetzten Thrombozyten von Spendern stammen, die bezüglich ihrer HLA-Antigene und thrombozyten-spezifischen Antigene bereits typisiert wurden. Mit den Thrombozyten verschiedener typisierter Spender kann auf einer Mikrotiterplatte ein ganzes Pannel, bezüglich der antigenen Strukturen verschiedener Thrombozyten in lyophilisierter Form aufgebracht und vorrätig gehalten werden. Das Reaktionsmuster des Patientenserums mit einem solchen Thrombozytenpannel lässt dann die Definierung der Patienten-Antikörper zu.

Diese Aufgabe wird erfindungsgemäss vorzugsweise dadurch gelöst, dass Thrombozytenmembranen durch einen Coating-Puffer und Zentrifugation an eine Polystyrolplatte gebunden werden. Eine spätere unspezifische Proteinadhäsion wird durch eine anschliessende Inkubation der Platte mit einer Gelatine-Blocklösung ausgeschlossen. Auf die so vorbereitete Platte wird das zu untersuchende Serum aufgetragen. Nach einer sich anschliessenden Inkubation wird die Platte mit 0,9 % NaCl-Lösung gewaschen. Dann werden den Kavitäten Anti-Human-Globulin-beschichtete Indikator-Latex-partikel (0,5 %ige Suspension) zugesetzt und zentrifugiert. Bei Anwesenheit von Antikörpern im Serum binden diese an die antigenen Strukturen der Thrombozytenmembranen. Die zugesetzten Indikator-Latex-Partikel zeigen dies an, indem das auf ihnen befindliche Anti-Human-Globulin an die bereits an Thrombozytenmembranen gebundenen Serumsantikörper bindet, was in einer gleichmässigen Wandadhärenz der Indikator-Latex-Partikel resultiert.

Beim Fehlen von Serumsantikörpern existieren für das Anti-Human-Globulin auf den Indikator-Latex-Partikeln keine Bindungsstellen, weshalb die Indikator-Latex-Partikel bei der Zentrifugation zu Boden der Kavität sinken und ein Sediment in Form eines konzentrierten Knopfes bilden.

Die Auswertung des Testes erfolgt visuell (über einer Lichtquelle): die Kavitäten der Platte werden auf Wandadhärenz bzw. Sedimentation der Latex-Partikel geprüft.

Wichtig ist das Mitführen einer negativen Kontrolle in Form eines antikörperfreien AB-Serums. Der positive Kontrollnachweis erfolgt durch ein Anti-Thrombozyten-Immunoglobulin der Klasse IgG in Verdünnung 1:640 der Fa. Dakopatts, Hamburg (Thrombocyte^{R}).

Der Test hat eine hohe Nachweisempfindlichkeit und erfasst Antikörper auch in niedriger Konzentration.

Die positive Reaktion ist einfach abzulesen und erfordert keine Erfahrung. Der Test ist in kurzer Zeit durchführbar (weniger als 2 h), das Ergebnis bleibt stabil und über viele Tage hinweg ablesbar.

Die Verwendung von Latex-Partikeln ist bisher häufig für Agglutinationstests beschrieben worden. Als Ersatz von Indikatorerythrozyten sind sie bisher unbekannt.

Das erfindungsgemässe Testprinzip unter Verwendung von Indikator-Latex-Partikeln ist auch zum Nachweis von Antikörpern gegen andere Zelloberflächenantigene, wie z.B. Antikörper gegen Erythrozytenmembranantigene geeignet.

Nachfolgend wird die Erfindung im einzelnen durch ein Beispiel erläutert.

### Beispiel

### I. Reagenzien und Geräte

Coating Puffer
Na-Carbonatpuffer 0,05 molar, pH 9,7
Stammlösung 1: Na₂CO₃ x 10 H₂O 28,62 g/l
Stammlösung 2: NaHCO₃ 8,4 g/l
Arbeitslösung: 40 ml Stammlösung 1 + 60 ml Stammlösung 2 + 100 ml H₂O dest.
Blocklösung (Blockpuffer)
Phosphatpuffer
0,01 molar, pH 7,2 Na₂HPO₄ x 2 H₂O 0,005 molar
NaH₂PO₄ x H₂0 0,005 molar
NaCl 0,4 molar
Gelatine 3,5 g/l
Physiologische Kochsalzlösung
0,9 % NaCl
Glycine Buffered Saline (GBS)
0,1 molar Glycine (Sigma Chemical Co. G 7126)
0,85 % NaCl, pH 8,2
Rinder-Albumin
fettsäurenfrei (Sigma Chemical Co. A 6003)
GBS/RSA
Glycine Buffered Saline mit Zusatz von 0,1 %
Rinderalbumin
Ethanol/Natriumacetat
95 %iger Ethanol mit Zusatz von 0,5 Gew.-% Natriumacetat
Polystyrol-Latex-Partikel der Grösse 11,9 µm Durchmesser, Dichte 1,05 als Suspension mit 10 % festen Bestandteilen (Sigma Chemicals Co. LB 120)
Affini Pure Goat Anti-Human-Immunglobulin
IgG + IgM (H+L) 2,5 mg/ml (Dianova Hamburg)
Thrombocyte^{R} als positive Kontrolle
RAH-IgG, Anti-Human-Thrombozyten vom Kaninchen, Verdünnung 1:640 (Dakopatts, Hamburg, Nr. 22502)
AB-Serum
Serum der Blutgruppe AB, getestet auf Antikörperfreiheit
Heparinblut
als Thrombozytenquelle, nach der Abnahme 2 - 3 h stehen lassen, dann thrombozytenreiches Plasma abnehmen, niemals zentrifugieren
Nativblut
als Serumquelle, bei 600 g, 5 min zentrifugieren
Mikrotiterplatten 96 Kavitäten (Greiner Nr. 650061) Wasserbad 37°C
Zentrifuge mit Einsätzen für Mikrotiterplatten.

### II. Herstellung der Indikator-Latex-Partikel

Die unbeschichteten Partikel bilden stabile hydrophobe Suspensionen, die, wenn sie mit einem Liganden (z.B. Antikörper) beschichtet werden, die Eigenschaften dieses Liganden annehmen und stabile lyophile Kolloide bilden. Sie bleiben in Suspension während der Lagerung. Sie aggregieren, wenn sie mit den Anti-Liganden (z.B. Antigen) zusammengebracht werden.

Die Liganden zur Beschichtung der Latex-Partikel können durch 3 verschiedene Methoden aufgebracht werden:

passive Adsorption, forcierte Adsorption und kovalente Bindung. Für die hier eingesetzten Latex-Partikel werden 2 Methoden vorgeschlagen: passive Adsorption und forcierte Adsorption.

### 1. Passive Adsorption

a) Herstellung einer 1 %igen Latex-Suspension mit Glycine Buffered Saline (GBS)
b) Zu 1 Volumenteil Latex-Suspension wird 1 Volumenteil Antikörperlösung (ca. 2 mg/ml) gegeben und gut gemischt c) Inkubation mindestens 30 min bei Raumtemperatur oder über Nacht bei 5°C (98 % des gesamten adsorbierten Liganden (Raumtemperatur) werden während der ersten 5 min adsorbiert, die restlichen 2 % während der folgenden 1 bis 2 h)
d) Zugabe 30 %iger Rinderserum-Albumin-Lösung (RSA), bis eine 1 %ige Endkonzentration an RSA erreicht ist
e) Inkubation 2 h bei 37°C und über Nacht bei 5°C oder 2-3 Tage bei 5°C
f) Zentrifugation der Mischung bei 1200 g, 5 min, um die Partikel zu präzipitieren
g) Aufnahme der Partikel in frisch bereiteter GBS/RSA + 0,05 % Natriumacid, Endkonzentration: 0,5 %ige Partikelsuspension.

### 2. Forcierte Adsorption

a) Zentrifugieren der Latexsuspension 5 min bei 1200 g
b) Ueberstand dekantieren (= Ueberstand A) und aufbewahren
c) Latexpartikel werden in 0,1 molarer GBS suspendiert (ca. 1 %ige Suspension)
d) Zugabe von Antikörperlösung, pro Volumenteil Suspension 1 Volumenteil Antikörperlösung (2 mg Protein/ml)
e) die Mischung wird 15 min auf dem Magnetrührer gerührt
f) pro Volumenteil dieser Mischung werden 10 Volumenteile einer Lösung von 95 %igem Ethanol + 0,5 Gew.-% Natriumacetat zugegeben, während die Mischung auf dem Magnetrührer gerührt wird g) Inkubation 30 min bei Raumtemperatur auf dem Magnetrührer
h) weitere Inkubation bei 5°C für die Dauer von 12-24 h
i) Zentrifugieren der Suspension bei 1200 g, 5 min
j) Ueberstand verwerfen
k) die Latexpartikel werden wieder im Ueberstand A resuspendiert und so lange bei Raumtemperatur gerührt, bis eine feine Suspension entstanden ist
l) pro Volumenteil Latexsuspension gibt man 1 Volumenteil GBS hinzu
m) pro 100 ml dieser (unter 1) bereiteten) Suspension gibt man 12,5 ml GBS/RSA zur Stabilisierung der Suspension
n) zur Vorbeugung vor bakterieller Kontamination kann man entsprechende Lösungen zusetzen
o) zum Einsatz im Test wird eine 0,5 %ige Latexsuspension eingesetzt.

### III. Herstellung von Thrombozyten-beschichteten Platten

1) Heparinblut nach der Abnahme 2-3 h stehen lassen, dann thrombozytenreiches Plasma mit Pasteurpipette (Glas) abnehmen
2) Vom Nativblut Serum abtrennen durch Zentrifugation
3) Pro Kavität der Mikrotiterplatte 100 µl des thrombozytenhaltigen Plasmas geben
4) Pro Kavität 100 µl Coating-Puffer (2 Tropfen) mit Pasteurpipette geben
5) Platte bei 1000 g 15 min zentrifugieren
6) Platte kräftig abkippen, 1-2 mal vorsichtig mit 0,9 % NaCl (aus Spritzflasche mit abgeschnittener Spitze) waschen, abkippen, leicht abtupfen
7) Pro Kavität 200 µl Blocklösung (Pasteurpipette) geben, 1 h bei 37°C im Wasserbad inkubieren
8) Platten kräftig abkippen, 2-3 mal mit 0,9 % NaCl waschen, abtupfen.

### IV. Testdurchführung

1) Die Platten aus III.8) werden sofort (nicht trocken werden lassen) mit den Kontrollseren bzw. dem Patientenserum bestückt. Pro Kavität gibt man 50 µl (1 Tropfen) Serum. Es sind dreifache Ansätze durchzuführen
2) Die Platten werden 30 min bei 37°C im Wasserbad inkubiert
3) Platten kräftig abkippen, 4-5 mal mit 0,9 % NaCl waschen und kräftig abkippen
4) Pro Vertiefung werden 50 µl (1 Tropfen) der Indikator-Latex-Partikel pipettiert
5) Die Platten werden bei 800 g 2 min zentrifugiert und über einer Lichtquelle abgelesen
6) Auswertung: positiv ist eine Vertiefung, wenn eine gleichmässige Adhärenz der Indikator-Latex-Partikel an der Wand der Kavität zu sehen ist. negativ ist eine Vertiefung, wenn die Indikator-Partikel zu Boden sinken und einen Knopf am Boden bilden.

## Patentansprüche

1. Verfahren zum Nachweis von in Blutserum enthaltenen Antikörper gegen antigene Strukturen auf Zellmembranen, unter Verwendung eines innen mit Zellmembranen beschichteten Gefässes, wobei das zu untersuchende Blutserum in das Gefäss gegeben wird, das beschickte Gefäss inkubiert wird, das Gefäss gewaschen, anschliessend eine Suspension von mit Anti-Human-Globulin beschichteten Indikatorpartikeln in das Gefäss gegeben wird und dieses einer Zentrifugation unterworfen wird, wobei beim Vorliegen von Antikörpern diese an die Zellmembranen gebunden werden, was zu einer gleichmässigen Wandadhärenz der Indikatorpartikel im Gefäss führt, während bei Abwesenheit der genannten Antikörper im Testserum die Indikatorpartikel nach der Zentrifugation im Gefäss ein Sediment bilden, dadurch gekennzeichnet, dass als Indikatorpartikel durch Anti-Human-Globulin beschichtete Latexpartikel verwendet werden.

2. Verfahren nach Anspruch 1, dadurch gekennzeichnet dass die Zellmembranen Thrombozytenmembranen sind.

3. Verfahren nach Anspruch 1 oder 2, dadurch gekennzeichnet, dass das Gefäss eine Kavität einer Mikrotiterplatte oder ein Teströhrchen ist.

4. Verfahren nach einem der Ansprüche 1 bis 3, dadurch gekennzeichnet, dass das Verfahren gleichzeitig mit mehreren Proben durchgeführt wird.

5. Verfahren nach einem der Ansprüche 1 bis 4, dadurch gekennzeichnet, dass die Zellmembranen mit einem Na-Carbonatpuffer, 0.05 molar, pH 9,7 und Zentrifugation an eine Gefäss aus Polystyrol gebunden werden.

6. Verfahren nach Anspruch 5, dadurch gekennzeichnet, dass eine anschliessende Inkubation mit Phosphatpuffer, ca. 0,01 molar, pH 7,2, und einem Gehalt von NaCl, 0,04 molar, und Gelatine 3,5 g/l, durchgeführt wird.

7. Verfahren nach einem der Ansprüche 1 bis 6, dadurch gekennzeichnet, dass die Inkubation bei ca. 37°C durchgeführt wird.

8. Verfahren nach einem der Ansprüche 1 bis 7, dadurch gekennzeichnet, dass das auf den Indikatorpartikeln vorhandene Anti-Human-Globulin durch passive Adsorption, forcierte Adsorption oder kovalente Bindung an die Latexpartikel gebunden ist.

## Claims

1. Procedure for detection in blood serum of antibodies against antigen structures on cell membranes using a vessel coated on the inside with cell membranes, the blood serum to be examined being put in the vessel, the coated vessel being incubated, the vessel being washed, then a suspension of test particles coated with anti-human globulin being put into the vessel and the vessel being subjected to centrifugation, whereby if antibodies are present they will bind to the cell membranes, which leads to an even wall adherence of the test particles in the vessel, while if said antibodies are absent in the test serum, the test particles form a sediment in the vessel after centrifugation, characterized in that used as test particles are latex particles coated with anti-human globulin.

2. Procedure according to claim 1, characterized in that the cell membranes are thrombocyte membranes.

3. Procedure according to claim 1 or 2, characterized in that the vessel is a cavity of a microtiter plate or a test tube.

4. Procedure according to one of the claims 1 to 3, characterized in that the procedure is carried out at the same time with several samples.

5. Procedure according to one of the claims 1 to 4, characterized in that the cell membranes are bound to a vessel of polystyrene with a Na-carbonate buffer, 0.05 molar, pH 9.7, and centrifugation.

6. Procedure according to claim 5, characterized in that the subsequent incubation is carried out with phosphate buffer, approx. 0.01 molar, pH 7.2, and a content of NaCl, 0.04 molar, and gelatine 3.5 g/l.

7. Procedure according to one of the claims 1 to 6, characterized in that the incubation is carried out at approx. 37° C.

8. Procedure according to one of the claims 1 to 7, characterized in that the anti-human globulin present on the test particles is bound to the latex particles through passive adsorption, forced adsorption or covalent binding.

## Revendications

1. Procédé pour la détection d'anticorps contenus dans du sérum sanguin à l'encontre des structures d'antigènes sur des membranes cellulaires, dans lequel on utilise un récipient dont l'intérieur est revêtu de membranes cellulaires, on met le sérum sanguin à examiner dans le récipient, on incube le récipient chargé, on lave le récipient, ensuite de quoi on y met une suspension de particules indicatrices enduites de globulines Anti-Human et on soumet celui-ci à une centrifugation, ces particules se liant aux membranes cellulaires en présence d'anticorps, ce dont résulte une adhérence uniforme des particules indicatrices aux parois du récipient, alors qu'en l'absence des dits anticorps dans le sérum de test les particules indicatrices forment un sédiment dans le récipient après la centrifucation, caractérisé en ce qu'on utilise comme particules indicatrices des particules de latex enduites de globulines Anti-Human.

2. Procédé selon la revendication 1, caractérisé en ce que les membranes cellulaires sont des membranes thrombocytes.

3. Procédé selon la revendication 1 ou la revendication 2, caractérisé en ce que le récipient est une cavité d'une plaque microtitre ou un tube de test.

4. Procédé selon l'une des revendications 1 à 3, caractérisé en ce que le procédé est exécuté en même temps avec plusieurs éprouvettes.

5. Procédé selon l'une des revendications 1 à 4, caractérisé en ce qu'on lie les membranes cellulaires à un récipient en polystyrol avec un tampon de carbonate de sodium, 0,05 molaire, pH 9,7 et centrifugation.

6. Procédé selon la revendication 5, caractérisé en ce qu'on effectue ensuite une incubation avec un tampon de phosphate, env. 0,01 molaire, pH 7,2 et un contenu de NaCI, 0,04 molaire, et 3,5 g/l de gélatine.

7. Procédé selon l'une des revendications 1 à 6, caractérisé en ce qu'on effectue l'incubation à une température d'environ 37°C.

8. Procédé selon l'une des revendications 1 à 7, caractérisé en ce qu'on lie les globulines Anti-Human qui se trouvent sur les particules indicatrices aux particules de latex par adsorption passive, par adsorption forcée ou liaison atomique.
